# FASCICULE DE BREVET EUROPEEN

(11) **EP 1 332 229 B1**
(45) Date de publication et mention de la délivrance du brevet: **30.12.2009**
(21) Numéro de dépôt: 01993389.4
(22) Date de dépôt: 08.11.2001
(51) Int. Cl.: C12Q 1/68, G01N 33/68, A61K 31/00, A61P 3/04

(54) **METHODE DE CRIBLAGE D'AGENTS SUSCEPTIBLES DE TRAITER L'OBESITE**
VERFAHREN ZUM SCREENEN VON MITTELN ZUR BEHANDLUNG DER FETTLEIBIGKEIT
METHOD FOR SCREENING AGENTS CAPABLE OF TREATING OBESITY

(30) Priorité: 10.11.2000 FR 0014487
(43) Date de publication de la demande: 06.08.2003
(73) Titulaire: Merck Sante, 69008 Lyon (FR); INSTITUT NATIONAL DE LA SANTE ET DE LA RECHERCHE MEDICALE (INSERM), 75013 Paris (FR)
(72) Inventeur: TARTARE-DECKERT, Sophie, F-06100 Nice (FR); VAN OBBERGHEN, Emmanuel, F-06100 Nice (FR)
(74) Mandataire: Bernasconi, Jean Raymond
(86) Numéro de dépôt international: PCT/FR2001/003473
(87) Numéro de publication internationale: WO 2002/038807

(56) Documents cités:
- WO-A-98/20112
- WO-A-98/29138
- JANDELEIT-DAHM K ET AL: "SPARC gene expression is increased in diabetes-related mesenteric vascular hypertrophy" MICROVASCULAR RESEARCH, vol. 59, no. 1, janvier 2000 (2000-01), pages 61-71, XP001010337
- TARTARE-DECKERT S ET AL: "The matricellular protein sparc/osteonectin as a newly identified factor up-regulated in obesity" JOURNAL OF BIOLOGICAL CHEMISTRY, vol. 276, no. 25, 22 juin 2001 (2001-06-22), pages 22231-7, XP001010354
- LEDDA M F ET AL: "SUPPRESSION OF SPARC EXPRESSION BY ANTISENSE RNA ABROGATES THE TUMORIGENICITY OF HUMAN MELANOMA CELLS" NATURE MEDICINE, NATURE PUBLISHING, CO, US, vol. 3, no. 2, 1 février 1997 (1997-02-01), pages 171-176, XP002061780 ISSN: 1078-8956
- GRAHAM J D ET AL: "Expression of Osteonectin mRNA in Human Breast Tumours is Inversely Correlated with Oestrogen Receptor Content" EUROPEAN JOURNAL OF CANCER, PERGAMON PRESS, OXFORD, GB, vol. 33, no. 10, septembre 1997 (1997-09), pages 1654-1660, XP004284400 ISSN: 0959-8049

## Description

La présente invention a pour objet une méthode de criblage d'agents susceptibles de traiter, de manière préventive ou curative, l'obésité ou l'un des désordres associés à l'obésité. Elle a également trait à une méthode de diagnostic de l'obésité ou de l'un des désordres associés à l'obésité en suivant l'expression de la protéine SPARC ou de son activité. Elle concerne une composition comprenant au moins un agent modulant l'expression ou l'activité de la protéine SPARC. Elle concerne aussi l'utilisation de tout agent modulant l'expression ou l'activité de la protéine SPARC pour la préparation d'une composition pharmaceutique destinée à traiter, de manière préventive ou curative, l'obésité ou l'un des désordres associés à l'obésité.

L'obésité résulte d'un déséquilibre positif de longue durée entre l'énergie emmagasinée et l'énergie dépensée. L'obésité est un désordre physiopathologique majeur qui peut être associé à de nombreuses maladies, telles que des désordres cardiovasculaires, incluant l'hypertension et l'artériosclérose, des désordres métaboliques, tels que le diabète, l'hyper insulinémie, l'insulino-résistance et certains types de cancer. Aux Etats-Unis, on estime que plus de 30 % de la population adulte est obèse, c'est à dire présente un poids de plus de 20 % au-delà du poids estimé normal. De nombreuses indications tendent à prouver que l'obésité est en train de devenir un problème de santé mondial.

On admet que le poids d'un corps est déterminé par des interactions multiples entre gènes et facteurs environnementaux, tels que régime alimentaire, état psychologique et fréquence d'activité physique. Il est reconnu que, pour de nombreux cas, un régime alimentaire et des exercices physiques ne suffisent pas pour perdre du poids, ceci est d'autant plus vrai chez les personnes prédisposées génétiquement à devenir obèse. De nombreux gènes semblent en effet contribuer à la pathogenèse de l'obésité. Ainsi, il a pu être récemment identifié des gènes spécifiques à l'obésité chez l'homme. Cependant, les mutations identifiées jusqu'à ce jour ne permettent d'expliquer qu'une proportion minime des syndromes de l'obésité.

Par conséquent, il s'avère nécessaire d'analyser l'expression spécifique de gènes dans le tissu adipeux pouvant expliquer les mécanismes contribuant à cette dérégulation chez les obèses. Les gènes présentant une expression altérée peuvent ainsi être une cible particulièrement adaptée pour le diagnostic ou le traitement, curatif ou préventif, de l'obésité.

C'est ainsi qu'après différentes études, les auteurs de cette invention ont trouvé que la protéine SPARC (Secreted Protein Acidic and Rich in Cysteine, également connue sous le nom d'ostéonectine et de BM-40) est sécrétée par l'adipocyte blanc, que son expression est plus élevée chez des modèles de souris "obèse" que chez des modèles de souris "maigre" et que son expression peut être régulée en fonction du régime administré.

La protéine SPARC est une glycoprotéine associée à la matrice extracellulaire largement distribuée dans les tissus humains en cours de développement. Elle est décrite comme régulant la morphogenèse, la prolifération et la différenciation cellulaire. Bien que son rôle spécifique reste encore incertain, son haut degré de conservation entre les espèces suggère une pression élevée pour la conserver lors de l'évolution. Dans la demande de brevet WO98/20112, il est décrit qu'une souris transgénique dépourvue du gène SPARC est enclin à l'hyperglycémie. Il y est également décrit une méthode de traitement de désordres associés à une sous-expression du SPARC, tels que notamment le diabète, la cataracte, l'ostéoporose et la protéinurie, en introduisant dans les cellules du mammifère atteint de ce désordre un polynucléotide codant pour la protéine. SPARC.

La présente invention a donc pour objet une méthode de criblage ***in vitro* ou conduite chez un mammifère non humain** d'agents susceptibles de **diminuer l'expression ou l'activité de la protéine SPARC chez un sujet obése ou prédisposé à l'obésité,** par modulation du taux d'expression ou de l'activité de la protéine SPARC.

Concernant la prévention de l'obésité, il peut en effet être intéressant de traiter à l'aide d'agents ainsi identifiés des sujets que l'on a diagnostiqués être prédisposés à l'obésité avant que ceux-ci ne présentent un poids trop élevé.

Plus spécifiquement, ce procédé de criblage comprend les étapes suivantes:
- mesurer les taux d'expression ou l'activité de la protéine SPARC en présence et éventuellement en l'absence de l'agent à tester,
- déterminer si le taux d'expression ou l'activité de la protéine SPARC est **diminuée** en présence de l'agent à tester par rapport à un témoin.
- éventuellement, identifier l'agent qui **diminue** le taux d'expression ou l'activité de la protéine SPARC.
   Soit l'agent à tester est directement administré par voie entérale ou parentérale au mammifère, généralement non humain, puis la mesure est alors réalisée dans ou à partir d'un matériel biologique dudit mammifère, tel qu'une cellule, un extrait cellulaire, un fluide corporel, du sérum, du plasma, un tissu ou un extrait de tissu. Soit l'agent à tester est mis en contact avec un extrait biologique, tel que notamment une cellule, un extrait cellulaire, un fluide corporel, du sérum, du plasma, un tissu, un récepteur ou un extrait de tissu et la mesure est alors réalisée directement, le prélèvement dudit extrait biologique ayant eu lieu avant ladite mise en contact.
   Ce taux d'expression ou cette activité peut être mesuré à partir d'un extrait biologique; tel que notamment une cellule, un extrait cellulaire, un fluide corporel, du sérum, du plasma, un tissu, un récepteur ou un extrait de tissu. Bien entendu, l'extrait ou le matériel utilisé doit être capable d'exprimer ou de répondre à une activité de la protéine SPARC. De préférence, l'extrait ou le matériel utilisé vient d'un tissu adipeux ou d'un fluide corporel, tel que sérum ou plasma. Par exemple, dans le cas des cellules, on préfère mesurer l'expression
ou l'activité de la protéine SPARC dans des adipocytes ou des cellules de muscles striés. Cette mesure peut donc nécessiter au préalable de prélever l'organe ou une partie de l'organe du mammifère à tester. De préférence, l'organe ou la partie de l'organe prélevé comprend un tissu adipeux. Plus particulièrement, le tissu adipeux est du tissu adipeux abdominal ou du muscle squelettique.

Selon l'étape suivante, on détermine si le taux d'expression ou l'activité de SPARC est modulée. Ainsi, on peut analyser s'il est augmenté ou diminué en présence de l'agent à tester par rapport au même test en l'absence de l'agent à tester (témoin). Cette modulation peut être également déterminée dans le temps. Ainsi, l'agent à tester peut être, par exemple, ajouté aux cellules
ou administré à un animal et des échantillons sont prélevés successivement dans le temps pour déterminer l'évolution dans le temps de l'expression ou de l'activité de la protéine SPARC. Dans ce cas, le témoin correspond à une mesure du taux d'expression ou de l'activité de la protéine SPARC en présence de l'agent, mais réalisée à un moment différent.

L'expression ou l'activité associée à la protéine SPARC peut être modulée de différentes manières. La modulation de son expression peut correspondre à la modulation du taux d'expression de la protéine SPARC ou de son gène. L'activité de la protéine SPARC correspond plus particulièrement à son activité biologique propre ou à sa signalisation, c'est à dire aux conséquences biologiques que son activité biologique entraîne. La modulation de sa signalisation peut correspondre à la modulation d'un effet biologique dû à la protéine SPARC, notamment en bloquant un site actif de la protéine SPARC
ou en modifiant sa conformation modulant ainsi un de ses effets biologiques.

Ainsi, les méthodes de mesure utilisables sont nombreuses et sont celles connus de l'homme du métier. On peut notamment citer la méthode ELISA (mesurant le taux d'expression de la protéine), les méthodes d'analyse Northern blot ou de PCR quantitative (mesurant le taux d'expression d'ARNm), les méthodes d'analyses biochimiques par mesure de la prolifération ou de la différenciation cellulaire, ou par analyse microscopique sur la morphogénèse du tissu étudié (dans ce cas, une corrélation entre la morphogénèse du tissu, matériel ou extrait biologique, et le taux d'expression ou l'activité de la protéine SPARC est à faire antérieurement).

De préférence, on mesure l'expression ou l'activité de la protéine SPARC circulante ou du tissu adipeux.

On définit, si nécessaire, la nature de l'agent qui module selon le procédé de la présente invention l'expression ou l'activité de la protéine SPARC.

Les auteurs de la présente invention ont donc pu mettre en évidence que l'expression de la protéine SPARC est plus élevée chez des modèles de souris "obèse" que chez des modèles de souris "maigre". Compte tenu de ces résultats, il est possible d'établir une relation entre des agents susceptibles de traiter l'obésité et leur capacité à diminuer l'expression ou l'activité de la protéine SPARC. Cependant, il est possible, comme dans le cas de la leptine qui peut être surexprimée chez les personnes obèses, d'envisager un traitement utilisant la protéine ou un agent augmentant son expression en tant qu'agent thérapeutique.

Ainsi, l'agent qui augmente ou qui diminue le taux d'expression ou l'activité de la protéine SPARC peut être utilisable pour traiter l'obésité ou l'un des désordres associés à l'obésité, l'obésité ou l'un des désordres associés à l'obésité présentant une surexpression de la protéine SPARC.

Plus particulièrement, ces agents ainsi identifiés sont susceptibles de traiter, de manière préventive ou curative, l'obésité ou l'un des désordres associés à l'obésité, tels que notamment l'hypertension, l'artériosclérose, le diabète (généralement le diabète de type 2), l'hyper insulinémie et l'insulino-résistance, ou plus globalement le Syndrome Métabolique d'Insulino-Résistance (SMIR). De préférence, ces agents sont susceptibles de traiter, de manière préventive ou curative, l'obésité.

De préférence, l'expression ou l'activité de la protéine SPARC correspond à celle de la protéine SPARC circulante ou du tissu adipeux. Ainsi, la protéine SPARC devient un outil de diagnostic.

Cette méthode peut permettre de suivre l'évolution de l'un de ces désordres. En effet, il peut s'avérer necessaire lors d'un traitement pharmaceutique de suivre l'évolution de l'un de ces désordres par le biais du suivi du niveau d'expression ou d'activité de la protéine SPARC.

Ainsi, le témoin peut être le matériel à diagnostiquer mais dont la mesure de l'expression ou de l'activité de la protéine SPARC est réalisée avant ou après la mesure de l'expression ou de l'activité de la protéine SPARC du matériel à diagnostiquer. On a alors une évolution dans le temps de l'expression ou de l'activité de la protéine SPARC. Le témoin peut être un être humain ne présentant pas l'un des ces désordres. On a alors une comparaison directe des taux d'expression ou de l'activité de la protéine SPARC d'un être humain ne présentant pas l'un des ces désordres et d'un être humain à diagnostiquer. Ainsi, si on observe une augmentation du taux d'expression ou d'activité de la protéine SPARC sur ledit matériel à diagnostiquer, ledit matériel provient d'un être humain obèse ou du moins prédisposé à l'être ou d'un être humain qui présente un des désordres associés à l'obésité. A partir de ces résultats, en réalisant ce test plusieurs fois de manière espacée dans le temps, on peut suivre l'évolution dans le temps d'un de ces désordres diagnostiqués, notamment dans le but de suivre l'efficacité ou l'inefficacité d'un traitement suivi par cette personne.

Le matériel à diagnostiquer peut être un des extraits biologiques prélevés, tels que décrits précédemment, d'un être humain. L'extrait peut donc être une cellule, un extrait cellulaire, un fluide corporel, du sérum, du plasma, un tissu ou un extrait de tissu. Bien entendu, le matériel ou l'extrait utilisé doit être capable d'exprimer ou de répondre à une activité de la protéine SPARC.

La mesure de l'expression ou de l'activité de la protéine SPARC est telle que décrite antérieurement.

L'obésité et les désordres liés à l'obésité sont plus particulièrement liés à une surexpression de la protéine SPARC.

Par voie entérale, on entend plus particulièrement la voie orale ou rectale. Par voie parentérale, on entend plus particulièrement une injection ou une application topique sur la peau, notamment au moyen d'un patch.

De préférence, l'agent module l'expression ou l'activité de la protéine SPARC circulante ou dans le tissu adipeux.

Des exemples concrets mais non limitatifs de l'invention vont maintenant être présentés.

### EXEMPLES

- Pour détecter les changements d'expression d'ARNm dans le tissu adipeux blanc du modèle de souris obèse GTG, le clonage par hybridation soustractive et PCR suppressive a été réalisé en utilisant, selon le protocole du fabricant, le kit de soustraction d'ADNc PCR-select (de la société Clontech, Palo Alto, CA, USA).

Le modèle de souris "goldthioglucose" (GTG) a été obtenu par administration en double injection de "goldthioglucose" dans une souris mâle OF-1 (de la société Iffa-Credo) âgée de trois semaines, tel que décrit dans la publication de Marchand-Brustel Y. et coll., (1978) Am. J. Physiol. 234: E348-358.

Des souris mâles OF-1 sont utilisées à titre de comparaison.

On utilise 2µg de ARNm du tissu adipeux blanc épididymaire de 4 souris "obèses" GTG (poids 60 ± 3g) en tant que "tester" et 2µg de ARNm du tissu adipeux blanc épididymaire de 8 souris OF-1 "maigres" (poids 35 ± 2g) en tant que "driver".

Après hybridation, des transcrits différentiels ont été sélectivement amplifiés par PCR-suppressive, tel que décrit dans la publication de Diatchenko, L. et al., (1999). Methods Enzymol. 303 : 349-80. Les produits PCR ont été sous-clonés dans le vecteur pCR 2.1 en utilisant le kit de clonage TOPO TA (Invitrogen, Groningen, Pays-Bas). Des clones transformés ont été sélectionnés au hasard et les plasmides ont été purifiés en utilisant des colonnes QIAprep (Qiagen).

Des séquences d'ADNc partiel ont été déterminées et comparées à l'aide des séquences disponibles sur la base de données GenBank et des bases de données d'EST (expressed sequence tag) de souris en utilisant le programme de recherche d'homologie BLAST.

Ainsi, cette analyse a révélé une séquence correspondant précisément à celle de la région 3' non traduite du gène SPARC de souris (voir Mason, I.J. et al., (1986) EMBO J. 5 : 1465-1472).

La sonde d'ADNc de pleine longueur codant pour la protéine SPARC de souris a été obtenue par reverse-transcription (RT-PCR) à partir du tissu adipeux blanc en utilisant le kit RT-PCR en une étape Super-Script selon les recommandations du fabricant (Life Technologies).

- Une hybridation Northern avec une sonde d'ADNc de pleine longueur codant pour la protéine SPARC a confirmé le taux élevé du transcrit majeur SPARC (2,2 kb) dans le tissu adipeux blanc de la souris GTG "obèse", ce taux étant 5 à 6 fois plus élevé que dans la souris "maigre" OF-1.

- L'expression d'ARNm de SPARC a également été examinée dans le foie et le muscle squelettique, deux autres tissus répondant à l'insuline et impliqués dans l'homéostasie du glucose.

Dans les deux types de souris, aucune expression de SPARC n'a été observée dans le foie. A l'inverse, de l'ARNm a été détecté dans les muscles mais à un niveau plus bas que dans le tissu adipeux. Cependant, la quantité exprimée d'ARNm de SPARC dans ce tissu était équivalente dans les deux types de souris.

L'expression de SPARC dans différents tissus de souris "maigre" a également été examinée. Il s'avère que le tissu adipeux brun exprime des ARNm de SPARC et qu'un des tissus majeurs qui exprime les transcrits de SPARC dans la souris adulte est le tissu adipeux blanc.

- L'expression d'ARNm de SPARC a été également étudiée chez la souris *ob*/*ob* et son contrôle "maigre" (respectivement souris mâle C57BL/6OIaHsd *oblob* et souris mâle C57BL/6OIaHsd +/?, âgées de 4-10 semaines de Harlan France).

La souris *ob*/*ob* produit une leptine non fonctionnelle et présente comme conséquences une hyperphagie, une obésité importante, une hyperinsulinémie et une insulino-résistance.

Une analyse Northern blot, réalisée selon des protocoles standards, de l'ARN total du tissu adipeux blanc de souris "obèse" *ob*/*ob* et de souris "maigre" hybridé avec une sonde d'ADNc de pleine longueur codant pour la protéine SPARC a révélé deux espèces d'ARNm de 4 et 2,2 kb, le dernier étant le principal messager.

Chez la souris *ob*/*ob*, les deux transcrits ont été induits approximativement 4 fois plus que chez la souris "maigre" de contrôle. Ceci démontre que l'expression du gène SPARC est élevée dans le tissu adipeux de la souris *ob*/*ob.*

Ces résultats montrent que la modulation de l'ARNm de SPARC dans le tissu adipeux n'est pas limitée à la forme acquise de l'obésité, telle que dans le modèle GTG, et que cette expression élevée ne dépend pas d'une signalisation intacte de la leptine.

- A côté des adipocytes, le tissu adipeux contient différents autres types cellulaires, incluant des préadipocytes, des cellules endothéliales, des cellules du muscle lisse, des fibroblastes, des mastocytes et des macrophages.

Pour déterminer la source d'expression de la protéine SPARC dans le tissu adipeux, des expériences de séparation cellulaire ont été réalisées.

Pour ce faire, des tissus épididymaires ont été prélevés à partir de souris *oblob* et de souris "maigre" (+/?), puis traités par une collagénase (Liberase Blendzyme 3 de Roche Molecular Biochemicals, Indianapolis), puis soumis à une centrifugation différentielle pour séparer les cellules adipeuses (surnageant) des cellules non-adipeuses (fraction stroma vasculaire dans le culot).

L'ARN total a été isolé en utilisant le TRlzol selon les instructions du fabricant (Life Technologies), l'ARN est quantifié en mesurant l'absorbance à 260 nm.

La quantité d'ARNm de SPARC associée à chacune de ces fractions cellulaires a été déterminée par RT-PCR.

Les résultats montrent que la majeure partie de l'ARNm de SPARC se trouve dans la fraction des adipocytes et que cette expression est plus élevée dans les adipocytes des souris "obèses" que dans celles des souris "maigres".

L'ARNm de SPARC a aussi été détectée dans la fraction stroma vasculaire, mais dans une moindre mesure. L'expression d'ARNm de SPARC et son augmentation dans le tissu adipeux des souris "obèses" se trouvent donc principalement dans les adipocytes.

- Pour mieux appréhender la modulation de la protéine SPARC dans l'obésité, l'expression du gène SPARC dans un modèle d'obésité induit par un régime particulier a été étudiée.

Une étude du développement de l'obésité a été réalisée avec une souche AKR prédisposée à l'obésité en réponse à un régime à teneur en graisses élevée.

Ces souris AKR/OIaHsd (West et al., (1992). Am. J. Physiol. 262 : R1025-R1032) âgées de 5 semaines reçoivent soit un régime contenant 12% de calories venant de graisses, soit un régime à teneur élevée en graisses (Teklad Adjusted Calories TD 97363, Harlan Teklad, Madison WI) contenant 21 % en poids de lipides de lait anhydre, ce qui correspond à 42% de calories venant de graisses.

Pendant 10 semaines de régime à teneur en graisses élevées, ces souris ont été pesées deux fois par semaine. Après 3, 6 et 10 semaines de régime, les souris ont été euthanasiées et les parties grasses épididymaires ont été immédiatement retirées, pesées et traitées pour extraire l'ARN total. L'ARN total a été isolé en utilisant le TRlzol selon les instructions du fabricant (Life Technologies), l'ARN est quantifié en mesurant l'absorbance à 260 nm.

A poids égal au départ, après 10 semaines, les souris ayant suivi un régime à taux élevé en graisses ont pris 15,3 ± 1,5 g, les autres souris ayant suivi un régime normal ont pris 10,5 ± 1,4 g. En outre, le poids des parties grasses épididymaires était quatre fois plus élevé chez les souris ayant suivi un régime à teneur élevée en graisses que chez celles ayant suivi un régime normal.

Par analyse Northern blot, après dix semaines de ces régimes, le taux du transcrit majeur de SPARC était trois fois plus élevé chez les souris ayant suivi un régime à teneur élevée en graisses que chez celles ayant suivi un régime normal.

En parallèle, a été étudiée l'expression du gène SPARC en fonction du temps, après 3, 6 et 10 semaines de régime à teneur élevée en graisses. Entre 3 et 6 semaines, le taux d'ARNm de SPARC a augmenté de manière significative. Après 10 semaines, le taux d'ARNm de SPARC avait tendance à diminuer, mais restait plus élevé que celui observé après trois semaines du même régime.

Ainsi, ceci montre que le taux d'ARNm est augmenté dans un modèle d'obésité obtenue par régime spécifique et cette augmentation arrive assez tôt dans le développement de l'obésité.

L'augmentation de l'expression du gène SPARC dans le tissu adipeux est caractéristique de l'obésité génétique ou acquise, induite par hyperphagie ou par un régime à teneur élevée en graisse.

Par analyse immunoblot, il a été constaté que le taux de protéine SPARC dans les adipocytes de souris "obèses" *ob*/*ob* est 3 à 4 fois plus élevé que chez la souris "maigre". Ces résultats démontrent que cette augmentation de protéine est en accord avec l'augmentation de l'expression de son gène.

- SPARC est une molécule sécrétée, une analyse *ex vivo* de la capacité des adipocytes à sécréter la protéine SPARC a été réalisée.

La concentration d'antigène SPARC dans un milieu conditionné pendant 16 heures d'adipocytes de rats fraîchement isolés a été mesurée par ELISA spécifique à SPARC. Une quantité sept fois plus élevée environ de protéine SPARC a été observée dans le milieu conditionné comparé au milieu de contrôle.

Ainsi, les cellules adipeuses matures produisent et sécrètent la protéine SPARC.

Par ailleurs, il a été trouvé que l'insuline injectée à des souris augmente l'expression du messager de la protéine SPARC dans le tissu adipeux.

- Comme l'expression élevée de SPARC dans les adipocytes est probablement associée à l'augmentation de la masse adipeuse dans l'obésité, une étude sur l'expression de SC1, une protéine liée à la protéine SPARC, a été réalisée pour voir si cette expression était également modulée dans le tissu adipeux de souris "obèses".

Les protéines SPARC et SC1 (connu aussi sous le nom de hevin) proviennent d'une petite famille de protéines qui sont définies par un domaine N-terminal variable, suivi de deux domaines conservés, un domaine comme la follistatine et un domaine liant le calcium. La protéine SC1 présente une grande similarité avec la protéine SPARC (70% de similarité au niveau des acides aminés) et a été envisagée pour compenser fonctionnellement le déficit en protéine SPARC dans les souris transgéniques dépourvues du gène SPARC.

Ainsi, suite à une analyse par RT-PCR, et contrairement à l'ARNm de SPARC, l'expression d'ARNm de SC1 chez les souris *ob*/*ob* et GTG "obèses" n'est pas augmentée dans ces modèles. Ceci montre que l'expression adipocytaire altérée de SPARC a des conséquences fonctionnelles spécifiques dans l'obésité.

## Revendications

1. Méthode de criblage in vitro ou conduite chez un mammifère non humain d'agents susceptibles de diminuer l'expression ou l'activité de la protéine SPARC chez un sujet obèse ou prédisposé à l'obésité. comprenant les étapes suivantes:
- mesurer les taux d'expression ou l'activité de la protéine SPARC en présence et éventuellement en l'absence de l'agent à tester,
- déterminer si l'expression ou l'activité de la protéine SPARC est diminuée en présence de l'agent à tester par rapport à un témoin,
- éventuellement, identifier l'agent qui diminue l'expression ou l'activité de la protéine SPARC.

2. Méthode selon la revendication 1, **caractérisée en ce que** l'agent à tester est directement administré par voie entérale ou parentérale au mammifère, puis la mesure est réalisée dans ou à partir d'un matériel biologique dudit mammifère.

3. Méthode selon la revendication 1, **caractérisée en ce que** l'agent à tester est mis en contact avec un extrait biologique et la mesure est alors réalisée directement, le prélèvement dudit extrait biologique ayant eu lieu avant ladite mise en contact.

4. Méthode selon l'une des revendications précédentes, **caractérisée en ce que** l'extrait ou le matériel utilisé vient d'un tissu adipeux ou d'un fluide corporel, tel que sérum ou plasma.

5. Méthode selon la revendication précédente, **caractérisée en ce que** le tissu adipeux est du tissu adipeux abdominal ou du tissu adipeux du muscle squelettique.

6. Méthode selon l'une des revendications précédentes, **caractérisée en ce que** l'expression ou l'activité de la protéine SPARC est l'expression de la protéine SPARC ou de son gène.

7. Méthode selon l'une des revendications précédentes, **caractérisée en ce que** l'on mesure l'expression ou l'activité de la protéine SPARC circulante ou du tissu adipeux.

8. Méthode selon l'une des revendications précédentes, **caractérisée en ce que** la méthode de mesure utilisée est choisie parmi la méthode ELISA, une méthode d'analyse Northern blot ou de PCR quantitative, une méthode d'analyse biochimique par mesure de la prolifération ou de la différenciation cellulaire, et une analyse microscopique sur la morphogénèse du matériel ou extrait biologique étudié.

## Claims

1. Method carried out in vitro or on a non-human mammal for screening agents capable of reducing the expression or activity of the protein SPARC in an obese subject or a subject predisposed to obesity, which method comprises the following steps:
- measuring the expression levels or activity of the protein SPARC in the presence and optionally in the absence of the agent to be tested,
- determining whether the expression or activity of the protein SPARC is reduced in the presence of the agent to be tested relative to a control,
- optionally identifying the agent that reduces the expression or activity of the protein SPARC.

2. Method according to claim 1, **characterised in that** the agent to be tested is administered to the mammal directly by the enteral or parenteral route and then the measurement is carried out in or starting from a biological material of said mammal.

3. Method according to claim 1, **characterised in that** the agent to be tested is brought into contact with a biological extract and then the measurement is carried out directly, said biological extract having been taken before being brought into contact with the agent to be tested.

4. Method according to any one of the preceding claims, **characterised in that** the extract or the material used comes from an adipose tissue or a body fluid, such as serum or plasma.

5. Method according to the preceding claim, **characterised in that** the adipose tissue is abdominal adipose tissue or skeletal muscle adipose tissue.

6. Method according to any one of the preceding claims, **characterised in that** the expression or activity of the protein SPARC is the expression of the protein SPARC or of its gene.

7. Method according to any one of the preceding claims, **characterised in that** the expression or activity of circulating SPARC protein or of the adipose tissue is measured.

8. Method according to any one of the preceding claims, **characterised in that** the measuring method used is selected from the ELISA method, a Northern blot analysis or quantitative PCR method, a method of biochemical analysis by measuring cell proliferation or differentiation, and microscopic analysis of the morphogenesis of the material or biological extract under study.

## Patentansprüche

1. Verfahren, um in vitro oder durch Ausführung bei einem nicht menschlichen Säugetier Wirkstoffe, die die Expression oder die Aktivität des Proteins SPARC bei einem fettleibigen oder zur Fettleibigkeit neigenden Wesen verringern können, zu klassifizieren, das die folgenden Schritte umfasst:
- Messen des Anteils der Expression oder der Aktivität des Proteins SPARC in Gegenwart und eventuell bei Abwesenheit des zu testenden Wirkstoffs,
- Bestimmen, ob die Expression oder die Aktivität des Proteins SPARC in Gegenwart des zu testenden Wirkstoffs in Bezug auf ein Kontrolltier abnimmt,
- eventuell Identifizieren des Wirkstoffs, der die Expression oder die Aktivität des Proteins SPARC verringert.

2. Verfahren nach Anspruch 1, **dadurch gekennzeichnet, dass** der zu testende Wirkstoff direkt enteral oder parenteral bei dem Säugetier verabreicht wird und dass dann die Messung in oder ausgehend von einem biologischen Material des Säugetiers ausgeführt wird.

3. Verfahren nach Anspruch 1, **dadurch gekennzeichnet, dass** der zu testende Wirkstoff mit dem biologischen Extrakt in Kontakt gebracht wird und dass dann die Messung direkt ausgeführt wird, wobei die Entnahme des biologischen Extrakts vor der Herstellung des Kontakts erfolgt ist.

4. Verfahren nach einem der vorhergehenden Ansprüche, **dadurch gekennzeichnet, dass** der verwendete Extrakt oder das verwendete Material von Fettgewebe oder von einem Körperfluid wie etwa Serum oder Plasma stammt.

5. Verfahren nach dem vorhergehenden Anspruch, **dadurch gekennzeichnet, dass** das Fettgewebe Unterleibsfettgewebe oder Skelettmuskelfettgewebe ist.

6. Verfahren nach einem der vorhergehenden Ansprüche, **dadurch gekennzeichnet, dass** die Expression oder die Aktivität des Proteins SPARC die Expression des Proteins SPARC oder seines Gens ist.

7. Verfahren nach einem der vorhergehenden Ansprüche, **dadurch gekennzeichnet, dass** die Expression oder die Aktivität des zirkulierenden Proteins SPARC oder des Fettgewebes gemessen wird.

8. Verfahren nach einem der vorhergehenden Ansprüche, **dadurch gekennzeichnet, dass** das verwendete Messverfahren gewählt wird aus dem Verfahren ELISA, einem Northem-Blot- oder quantitativen PCR-Analyseverfahren, einem biochemischen Analyseverfahren durch Messen der Zellwucherung oder Zelldifferenzierung und einer mikroskopischen Analyse der Morphogenese des untersuchten Materials oder biolgischen Extrakts.
